# EUROPEAN PATENT SPECIFICATION

(11) **EP 1 351 720 B1**
(45) Date of publication and mention of the grant of the patent: **08.10.2008**
(21) Application number: 02728327.4
(22) Date of filing: 18.01.2002
(51) Int. Cl.: A61L 24/04, A61L 15/22, C08L 23/16

(54) **HYDROCOLLOID COMPOSITIONS**
HYDROKOLLOIDZUSAMMENSETZUNGEN
COMPOSITIONS HYDROCOLLOIDES

(30) Priority: 19.01.2001 US 262930 P; 17.01.2002 US 53387
(43) Date of publication of application: 15.10.2003
(73) Proprietor: Scapa North America, Windsor, CT 06095 (US)
(72) Inventor: DING, Jian Ling, Glastonbury, CT 06033 (US); D'AMATO, Ferdinando, J., Suffiend, CT 06078 (US)
(74) Representative: Petraz, Gilberto Luigi
(86) International application number: PCT/US2002/001588
(87) International publication number: WO 2002/072164

(56) References cited:
- EP-A- 1 033 141
- WO-A-98/54268
- WO-A-99/45977
- US-A- 5 750 136
- DATABASE WPI Section Ch, Week 200063 Derwent Publications Ltd., London, GB; Class A17, AN 2000-650558 XP002210617 & JP 2000 265017 A (NITTO DENKO CORP), 26 September 2000 (2000-09-26)

## Description

*Field of the Invention*

This invention relates to hydrocolloid compositions. In particular, the invention is concerned with a hydrocolloid composition which is absorbent, adhesive, maintains its integrity upon moisture absorption, and is non-damaging to the skin when used in skin applications.

*General Background and State* of *the Art*

Wound care is desirable to improve the health and appearance of underlying dermal tissues. Wounds, either injury induced, such as cuts, abrasions or blisters, or surgically induced, such as surgical incisions or ostomies, for example, require localized treatment to remedy the affected area and to prevent further dermal damage. If wounds are not properly treated, further dermal irritation can occur resulting in secondary infections and further discomfort to the patient.

Hydrocolloid dressings formed of hydrocolloid compositions are widely used in the area of wound and ostomy care due their beneficial effects to the healing process. Hydrocolloid compositions used for wound care and ostomy applications preferably have a high degree of absorbency to absorb fluid away from the wound site and thus facilitate the healing process, balanced adhesion to hold the dressing to the skin of the user but permit painless removal, good structural integrity to permit easy and complete removal of the dressing from the skin, and should not further aggravate the primary wound by causing further dermal irritation.

Several hydrocolloid compositions have been described, however, each has disadvantageous qualities.

The first hydrocolloid composition was descried in U.S. Patent 3,339,546. The hydrocolloid composition described contains a continuous phase of low to medium molecular weight polyisobutylene (PIB) having a discontinuous phase of hydrophilic particles. Such hydrocolloid composition provides dry adhesion to skin and high absorbency to biological fluids. The disadvantage of this composition is its low integrity when it absorbs a great amount of fluids due to the low cohesion of the PIB.

U.S. Patent 4,166,051 described a PIB composition having butyl rubber added as an integrity enhancer. In addition to butyl polymer, U.S. Patent 4,192,785 and U.S. Patent 4,204,540 also included fiber material and insoluble hydrophilic powders as integrity enhancers. U.S. Patent 4,496,357 described a PIB composition having fumed silica added to increase the cohesive strength of the composition. In each of the compositions above, the integrity and/or cohesion of the PIB composition was improved, however, the total absorbency of the compositions compared to the original PIB system was reduced.

Other improvements in hydrocolloid compositions were attempted to achieve a better balance of adhesion and integrity, while improving absorption. U.S. Patent 4,359,047 described PIB blends having polyols and maleic anhydride. U.S. Patent 4,393,080 described PIB blends having methyl vinyl ether and maleic anhydride. U.S. Patent 4,551,490 described PIB blends having butyl rubber and styrenic block copolymer adhesive containing rosin tackifiers. U.S. Patent 5,466,726 described PIB blends with styrene butyldiene polymer and halobutyl polymers to improve integrity by modifying PIB. U.S. Patent 5,492,943 described blends of high molecular weight PIB and styrene butyldiene block copolymer, tackifier and plasticizing oil. EP 122344 described blends of low molecular weight of PIB with maleic vinyl ether and maleic acid.

In each of the compositions above, the absorption of the composition was improved over the original PIB composition. However, these compositions all contained organic ingredients which were more chemically reactive than PIB, and therefore less useful in a physiological system, as the skin of users was more likely to be irritated by the chemicals added to the PIB.
Document WO-A-9854268 discloses a hydrocolloid composition comprising an ethylene propylene diene monomer polymer and a polyisobutylene, the composition further comprising hydrophilic particles.
Document WO-A-9945977 discloses hydrocolloid compositions comprising an ethylene propylene monomer polymer and an ethylene propylene elastomer, the composition further comprising hydrophilic particles.

Clearly there is a need for a hydrocolloid composition which provides enhanced absorbency and/or enhanced structural integrity and/or improved adhesion qualities and/or minimized skin damage to a user. This is achieved by a hydrocolloid composition having the features disclosed in claim 1. The depending claims outline advantageous forms of embodiment of a hydrocolloid composition according to the present invention. The hydrocolloid composition herein disclosed achieves at least the described properties.

### BRIEF DESCRIPTION OF THE DRAWINGS

FIG. 1 is a perspective view of an exemplary hydrocolloid dressing applied to skin.

FIG. 2 is a diagrammatic view of an exemplary method for manufacturing dressings, according to the teachings of the present invention.

### DETAILED DESCRIPTION OF THE PREFERRED EMBODIMENTS

While the specification describes particular embodiments of the present invention, those of ordinary skill can devise variations of the present invention without departing from the inventive concept and scope of the present invention.

Materials

According to the invention, there is provided a hydrocolloid composition comprising an ethylene propylene diene monomer polymer (EPDM). EPDM is a terpolymer made from ethylene, propylene and a non-conjugated diene, including, but not limited to ethylidene norbornene (ENB) or dicyclopentadiene (DCPD). Various embodiments may have ethylene/propylene ratio ranges from about 25% ethylene and 75% propylene to about 75% ethylene and 25% propylene. That is, the ethylene component of exemplary EPDMs ranges from about 25% to about 75%, and the propylene component ranges from about 25% to 75%. The diene content in the terpolymers like these typically ranges from about 1 % to about 20%. One example of EPDMs suitable for use in the invention is Royalene 521 (Uniroyal Chemical, Middlebury, CT, USA). Another exemplary EPDM suitable for use in the present invention is Royalene 512 (Uniroyal Chemical, Middlebury, CT, USA). This EPDM has a higher molecular'weight than Royalene 521, and provides a higher cohesive characteristic to the hydrocolloid composition.

These exemplary EPDMs exhibit various physical properties. The Royalene 512 has a Mooney Viscosity of about 60 at about 125 degrees centigrade and has a ethylene/propylene ratio of about 68/32. The exemplary diene content of this EPDM is about 4% ENB. The Royalene 521 has a Mooney Viscosity of about 45 at about 100 degrees centigrade and has a ethylene/propylene ratio of about 52/48. The exemplary diene content of this EPDM is about 5% ENB. Royalene 512 typically displays higher internal strength and shape retention, which provides improved cold flow resistance to exemplary hydrocolloid formulations. Alternatively, there is provided a hydrocolloid composition comprising an EPDM in combination with polyisobutylene (PIB) and ethylene propylene elastomers.

Ethylene/propylene elastomer is an amorphous polyolefin. Various embodiments have a Thermosel Viscosity at 190 degrees centigrade in the range of 250 to 20,000 mPas. Two suitable examples are Eastoflex E1003 and Eastoflex E1060 (Eastman Chemical, Kingsport, Tennessee, USA). The Eastoflex E1003 has a Thermosel Viscosity of 250 mPa s and a glass transition temperature of -33 degrees centigrade. In other embodiments, Eastoflex E1060 can be used which has a Thermosel Viscosity of 6,000 mPa s and a glass transition temperature of -23 degrees centigrade. Eastoflex E1060 typically displays higher tensile strength and percent elongation, which provides greater integrity to formulations. However, Eastoflex E1003 allows a better saline absorption.

In one embodiment, the EPDM/PIB ratio ranges from about 5% EPDM and 95% PIB to about 70% EPDM and 30% PIB. Examples of PIBs suitable for use in the invention are PIB 6H (Nippon Petrochemicals Co., Ltd., Tokyo, Japan) and Vistanex (Exxon Mobil Chemical Co., Houston, Texas, USA). In some embodiments, PIB grades can have average molecular weight in a range of 36000 to 70000. In other embodiments, low molecular weight to medium high molecular weight PIB can be used, such as Vistanex LM-MH (Flory Molecular Weight 50,400-55,800).

Additional exemplary hydrocolloidal compositions are comprised of EPDM in combination with PIB and styrenic block copolymers (SBC). SBCs are exemplary block copolymers made from polystyrene and polydiene blocks that are linked chemically. In the case of polydiene being a polyisoprene, the SBC is comprised of Styrene-Isoprene-Styrene tri-block and di-block polymers called SIS and SI (styrene isoprene). SBS represents a tri-block polymer with polybutadiene as the polydiene. That is, exemplary tri-block copolymer like SIS is composed of Polystyrene block-polyisoprene block- polystyrene block, which has three blocks. The block in the middle of this tri-block polymer is a polyisoprene called mid-block. Hence, a di-block, such as SI, can be Polystyrene block-Polyisoprene block linked together chemically. When the mid-block polydiene of a tri-block copolymer is hydrogenated, it becomes Styrene-Ethylene/Butylene-Styrene block copolymer called SEBS.

SIS, SBS and SEBS are compatible with EPDM rubbers. When blended with EPDM rubbers, they provide cohesive strength to the hydrophobic rubber pressure sensitive adhesive phase (PSA) of a hydrocolloid composition, reduce cold flow of the rubber phase, and promote skin adhesion.

SBCs which may be utilized in exemplary hydrocolloid compositions, according to the teachings of the present invention, include, but are not limited to, Kraton 1107, Kraton 1111 and Kraton 1101 of Shell Chemical, Vector 4113 and Vector 4114 of Exxon Mobil. Exemplary hydrocolloid compositions may contain up to 30% by weight of a hydrocolloid formulation). These are SIS based copolymers, except for Kraton 1101, which is a SBS based SBC.

Hydrophilic particles can be added to the composition and are preferably capable of swelling in water and transporting water. Hydrophilic particles which may be used in the invention include, but are not limited to naturally derived substances (such as silica, collagen, pectin, gelatin, starches, guar gum, gum arabic, xanthan gum, locust bean gum, gum karaya, alginic acid and its sodium or calcium salts) and synthetic substances (such as such as sodium carboxymethylcellulose (CMC), crosslinked sodium carboxymethylcellulose, crystalline sodium carboxymethyl cellulose, polyvinyl alcohol, polyvinyl pyrollidone, high molecular weight polyethylene glycols and polypropylene glycols, cross-linked dextran and starch-acrylonitrile graft copolymer, starch sodium" polyacrylate, gluten, polymer of methyl vinyl ether and maleic acid and derivatives; polyvinyl pyrrolidone, polyethylene glycols, polypropylene plycols, metal and/or ammonium salts of polyacrylic acid and/or its copolymers, and metal or ammonium salts of polystyrene sulfonic acid) or a variety of alternative commercially available absorbent products. In one embodiment, the hydrophilic particles comprise about from about 20-40% of the hydrocolloid composition.

In some embodiments, the composition can include therapeutic agents, including those which can assist with wound protection and healing, such as alcohol, peroxide or betadine; antimicrobials; antibacterials, such as Triclosan, or polysporin; antivirals, such as Nonoxyl-9; antifungals, such as imidazole; antinflamatories such as hydrocortizone; wound healing promoters, such as growth factors; collagen; moisturizers, such as aloe or vitamins A, D or E; anti-scaring medications such as cortisone or pharmacologically active agents, such as, analgesics; anesthetics, anti-inflammatory and steroids.

In some embodiments, the composition can include additives, such as tackifiers, plasticizers and/or stabilizers to achieve the desired adhesive properties. Butyl rubber, including, but not limited to Butyl 268 & 269 (Exxon Mobil Chemical Co., Houston, Texas, USA) can be used to improve the integrity of hydrocolloid, as discussed above. Parapol (Exxon Mobil Chemical Co., Houston, Texas, USA), a polybutene, Eastoflex E1003 or 1060, resins (Eastman Chemical, Kingsport, Tennessee, USA) and low molecular weight ethylene propylene copolymers like Trilene 56 from Uniroyal Chemical can be used as plasticizers. The tackifiers used can be any aliphatic type resins, preferably with a soft point in a range of about 60 degrees centigrade to about 120 degrees centigrade. Eastotac 100L (Eastman Chemical, Kingsport, Tennessee, USA), for example, may be used as a tackifier. Other tackifiers include, not limited to, typical C5 resins like Wingtac 95 from Goodyear (Akron, Ohio, USA), hydrogenated aromatic resins and polyterpene resins like Regalite R101 and Piccolyte S115 from Hercules Inc. (Wilmington, Delaware, USA), and cyclic reins such as Escorez 5000 series from Exxon Mobil Chemical Co. Escorez 1310L, another tackifier from Exxon Mobil Chemical Co., is useful for practicing the teachings of the present invention. Escorez 1310L, is a hydrocarbon based aliphatic resin made from C5 monomers.

Hydrocolloid Composition Preparation

The method of manufacturing for the composition can be achieved, but is not limited to, the method or order of operations as described below. The compositions in Tables 1, 2 and 3 were prepared as follows:

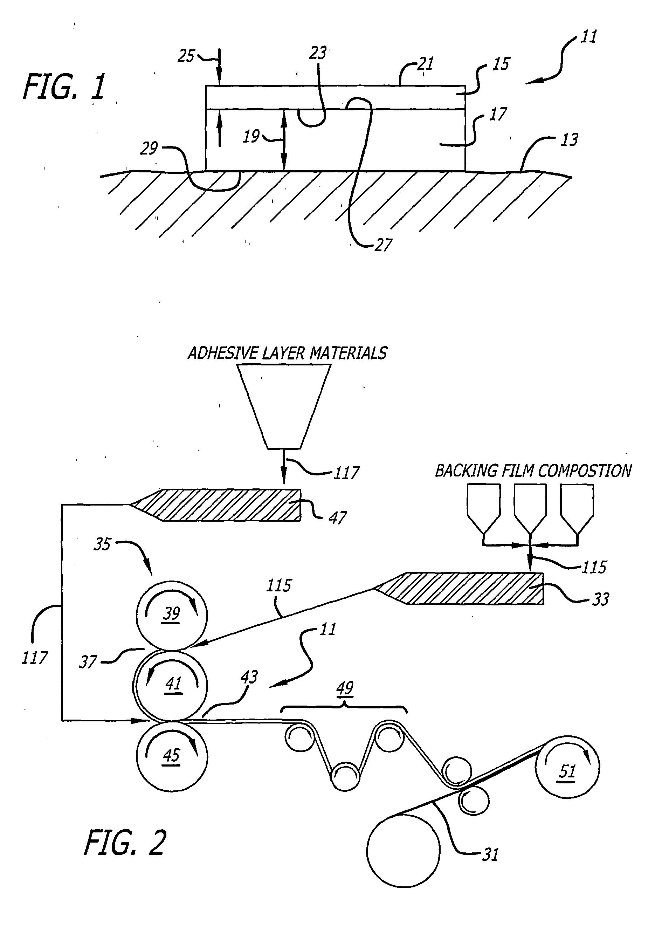

Ingredients including EPDM, PIB, rubber(s), and other ingredients, are added in a mixer and heated to about 180 to about 190 degrees centigrade under nitrogen blanket. The ingredients are heated until they are completely melted and the ingredients are mixed until the composition is homogeneous. Hydrophilic particles and therapeutic agents are then blended into the heated composition and mixing continued until the particles are mixed uniformly throughout. The composition can be extruded or pressed to a desired thickness, and then laminated to suitable substrates, such as backing film, release liner, polyurethane film, copolyester film, ethylene vinyl acetate (EVA) film, ethylene methyl acrylate copolymer (EMAC) film and polyolefine film, for example, to form sample sheets. The hydrocolloid samples of Table 1 through Table 3 were backed by a 5 mil EMAC film for absorption and skin adhesion tests. If the composition is thus laminated, the hydrocolloid dressing 11 , as seen in FIG. 1, is comprised of an adhesive layer 17 and backing layer 15. Alternatively, therapeutic agents may be added after cooling of the composition and/or after formation into dressings for use in wound care.

Exemplary dressings for which hydrocolloid compositions would be useful include ostomy adhesive assemblies, wound dressings, blister patches, bandages, other dressings and adhesive components of medical devices, as known to those of skill in the art. Such dressings may be cut into various shapes, such as oval, circle or round, rectangular, square and with stepped edge, beveled edge for general use or may be formed into desirable shapes for use on particular areas of a body, for example.

As known in the art, a hydrocolloidal dressing may comprise a backing film layer 15, such as a film or foam layer, for example, having an effective amount of adhesive material forming adhesive layer 17, comprised of a hydrocolloidal composition, bonded thereto. Exemplary material useful for such a backing film is further described below and may be utilized in calendered manufacturing processes, as known to those of skill in the art.

Backing Film Layer

FIG.1 depicts an exemplary hydrocolloid dressing 11, which includes a backing film layer 15 having an upper surface area 21 and a lower surface area 23, and constitutes a thickness 25; and an adhesive layer 17 adhered to the backing film layer lower surface area 23. The adhesive layer 17 has an upper surface area 27 and a lower surface area 29, and constitutes a thickness 19. Adhesive layer 17 is comprised of novel hydrocolloidal compositions formulated according to the teachings of the present invention. In use, the adhesive lower surface area 29 is adhered to the skin 13 of the user.

The backing film layer materials which are useful for this invention are not particularly limited as long as they can provide a suitable substrate for the adhesive layer 17 and are sufficiently strong to withstand removal from the skin 13 and maintain its integrity, having been secured to the skin 3 by the adhesive layer 17. Preferably, the backing film layer is water impervious.

The backing film layer 15 is preferably flexible from the viewpoint of comfort. The flexibility is achievable by elasticity in any one or all axes of the material. Further, the backing film layer 15 is preferably pliable to accommodate skin contours, when applied to areas of skin having alterations in surface angles. The backing is preferably non-stretchable, namely non-elastic, in the planar axis of the material.

The backing film layer of the hydrocolloid dressing 11 preferably includes a thermoplastic elastomer, and more specifically an ethylene based copolymer. Examples of ethylene based copolymers which may be used include, but are not limited to, ethylene acrylic acrylate, ethylene butyl acrylate, ethylene ethyl acrylate and ethylene methyl, acrylate copolymer (EMAC). In one embodiment, the backing film is preferably about 50% to about 100% ethylene based copolymer. The ethylene based copolymers used in the backing preferably have comonomer levels of about 8-28% and most preferably about 21 %. The levels of comonomer in the ethylene based copolymer of the backing film layer 15 can be selected to achieve a pliable backing which is comfortable for the user to wear. Further, the ethylene based copolymers used are preferably in the range of about 2 to about 10 melt index (MI) resins, however, as is appreciated by those skilled in the art, other grades of ethylene based copolymer could be used. Examples of ethylene based copolymer which can be used for the backing include, but are not limited to EMACs, such as Chevron SP2205, Exxon Optema^{®} TC-110 and Exxon Optema^{®} TC-120.

Further, the backing film layer 15 can include a low density polyethylene homopolymer (LDPE). The addition of LDPE may be advantageous at least in improving the processing speed by increasing the melt strength of a calendered film. A wide range of LDPEs can be used in the backing. The LDPEs used in the backing are preferably in the range of about 2 to about 16 MI extrusion and/or coating grade resins. Examples of LDPEs which can be used in the backing film layer 15 include, but are not limited to Nova Chemicals LF-0219-AM or Chevron PE1019.

The backing film layer 15 can further include additives, such as antioxidant/stabilizer and/or processing aids. Hindered phenolic antioxidant such as Irganox^{®} 1010 manufactured by Ciba-Geigy is an example of an appropriate stabilizer for medical applications. Processing aids such as N,N' Ethylene Bisstearamide may be advantageous at least in benefiting the processing by assisting in the release of the calendered film from a center roll surface. Use of processing aids is particularly preferable in backing film formulations where the comonomer level exceeds 18%. An example of one such additive is Acrawax^{®} C manufactured by Lonza Specialty Chemicals. In an alternate embodiment the backing film is a composition of about 65% to about 100% by weight EMAC, up to about 35% by weight LDPE, about 0.05 to about 2% by weight antioxidants, processing aids and/or stabilizers.

The backing film layer 15 is also preferably of a thickness 25 to provide sufficient strength to the dressing 11, but also of a thinness which will be comfortable to the wearer and pliable to contact all skin surfaces 3. In one embodiment, the backing film layer thickness 25 is about 0.5 to about 10 thousands of an inch (mils), and in other embodiments, the thickness of the backing film layer is about 2 to about 6 mils. The backing film layer thickness 25 may or may not be constant from the backing film upper surface area 21 to the backing film lower surface area 23.

In general, the foregoing hydrocolloidal compositions may be formed into sheets by passing the material through a calender having a pre-set gap, or the sheets may be formed by compression in a mold cavity of the desired depth. The compositions may also be formed by passing the mixed material through conventional extrusion equipment equipped with a slot or tape die set to extrude a ribbon of approximately the desired thickness. If necessary the extruded ribbon can be further compressed in thickness by passing it through one or more sets of compression rollers.

An exemplary mode of preparation comprises steps for extruding a ribbon of the desired width and thickness directly onto release paper, which is then cut into stock "preforms". If desired, the preforms can be covered, on their exposed sides, with one of several types of backings, either porous or non-porous films being used. In addition to backings previously disclosed, other available backings may be utilized, such as a plastic film, porous or non-porous, with or without a contact adhesive; non-woven as well as woven fabrics; porous or non-porous types of contact adhesive backed tapes. The backings may be applied by hand or in conjunction with the compression rollers referred to above.

Additional exemplary methods for the preparation of calendered dressings utilizing novel hydrocolloidal compositions, in accordance with the teachings of the present invention, may also be utilized. In one example, U.S. Patent Application Ser. No. 09/925,063, filed August 8, 2001 and incorporated herein in its entirety by reference, provides exemplary processes for the formation of hydrocolloidal dressings.

Method of Manufacture of the Hydrocolloid Dressing

The method of manufacturing for the dressing can be achieved, but is not limited to the method or order of operations as described below.

In one exemplary method of manufacturing hydrocolloid dressing 11,a, multi-roll calender process is used to form the backing film layer 15 and apply the adhesive layer 17 in a single manufacturing step (FIG. 2). The backing film composition 115 is formed by blending the selected polymers, antioxidants, processing aids and/or stabilizers in selected proportions that are metered, mixed and extruded, via a single screw extruder 33, for example. The temperature of the backing film composition 115 when extruded is preferably in the range of about 350-400 degrees Fahrenheit, and most preferably about 380 degrees Fahrenheit. The backing film composition 115 is delivered to the multi-rolled calender 35 in a continuous fashion for forming into the backing film layer 15. The backing film layer thickness 25 is determined by the width of the top gap 37 between the calender top roll 39 and center roll 41.

The calender top roll 39 surface temperature is preferably heated to the temperature of the backing film composition 115, while the center roll 41 is cooled relative to the temperature of the extrudate. Further, the top roll 39 preferably rotates at a slower rate relative to the center roll 41. The backing film composition 115 preferably sticks to the cooler and faster center roll 41 and is carried to the lower gap 43 between the calender center roll 41 and the lower roll 45 to be laminated with adhesive layer 17. The total thickness (at least the adhesive layer thickness 19 + the backing layer thickness 25) of the hydrocolloid dressing 11 is determined by the width of the lower gap 43 between the calender center roll 41 and lower roll 45.

Next, the adhesive composition 117 is prepared for extrusion on to the calender. As is appreciated by those skilled in the art, adhesives can be prepared in a number of ways and in a variety of mixing devices. For example, batch mixers (such as internal, sigma blade mixers including an AMK Mixtruder^{®}) can be used to mix the rubber-based adhesives prior to the calendering step. Secondary operations can be used to prepare the adhesive off-line when batch mixing is used. Alternatively, continuous mixers (such as a Farrel Continuous Mixer (FCM)^{®}) or twin screw extruders can be used. Continuous mixing typically allows for the adhesive can be mixed and fed to the calender directly. Preferably, the final delivery of the adhesive composition 117 to the calender 35 is accomplished by extrusion, with a single screw extruder 47 or melt pump system for example. The temperature of the adhesive composition 117 when, extruded is preferably in the range of about 400 - 450 degrees Fahrenheit, and most preferably about 430 degrees Fahrenheit.

The method of manufacturing for the adhesive composition can be achieved, but is not limited to the method or order of operations as described below. For example, adhesive composition ingredients may be comprised of EPDM and may be added in a sigma blade mixer and heated to about 180-190 degrees centigrade under nitrogen blanket. The ingredients are preferably heated until they are completely melted, and additionally the Parapol then added in some embodiments. The mixture is preferably mixed until the composition is homogeneous. The temperature is preferably reduced to about 110 - 130 degrees centigrade. Hydrophilic particles may then be blended into the heated adhesive composition and mixing continued until the particles are mixed uniformly throughout. The mixture is then discharged from the mixer and ready for calendering. An exemplary calender temperature may be in the range of about 130 to 160°C.

The adhesive composition 117 is calendered onto the backing film layer 15 between the center roll 41 and lower roll 45. A wide range of adhesive thickness 19 can be achieved by this method. The lower roll 45 is preferably heated to the temperature of the adhesive composition 117 when extruded. Further, the lower roll 45 preferably rotates at a slower rate relative to the center roll 41.

Once laminated, the hydrocolloid dressing 11 is stripped from the calender and preferably conveyed through a cooling section 49.

The adhesive lower surface 29 may then laminated with a release liner 31, and wound on to a master roll 51 for converting the hydrocolloid dressing material into individual use patches by cutting or pressing the dressings into the desired shapes and packing them for distribution to the user. A release liner 31 may, but need not be added, at any time after the product has been manufactured, for conversion or for distribution to the user (see FIG. 2).

Manufacturing can be carried out in the absence of a release liner. However, after the manufacture of the hydrocolloid dressing 11 a release liner 31 may be laminated to the adhesive layer lower surface area 29 to facilitate conversion of the dressing (such as by die cutting) or to protect the adhesive before application to the user, for example. Examples of suitable liner materials include, but are not limited to bleached Kraft-Glassine paper, silicone coated on one side at least where contact with the adhesive layer is made.

The liner 31 can be of the same dimensions as the hydrocolloid dressing 11, or can be of different dimensions to facilitate removal of the liner 31 from the dressing 11. Where the liner 31 is of different dimensions as the dressing, the liner can be larger in any one or all planar dimensions than the dressing 11. Further, the liner 31 can have lines of weakness, such as scores or perforations, so as to facilitate removal of the liner from the dressing.

The following studies (Tables 1, 2 and 3) and results were conducted in order to compare various characteristics of prior art hydrocolloidal compositions with the formulations disclosed herein, as well as provide exemplary innate absorption, integrity and adhesion characteristics.

Example 1

Saline Absorption Test - A sample of the hydrocolloid composition, on a backing of 5 mil EMAC film, was used to seal a beaker containing a fixed volume of saline. The beaker was inverted so that the saline covered the surface of the hydrocolloid composition. The testing set up was stored for 72 hours at 25 degrees centigrade and 50% relative humidity.

The percent of saline absorption was calculated from the weight gain of the hydrocolloid mass from the beginning of the test relative to that at the end of the test. The integrity of the sample after the test was inspected and rated on a scale of 1-10--a higher score indicating better integrity.

Skin Adhesion -A sample of the hydrocolloid composition was applied to the forearms of three subjects (1 female and 2 males). After six hours of continuous wear, the samples were removed and immediately evaluated for skin adhesion and physical irritation of the skin of the subject. Each observation was rated on a scale of 1-10--a higher score indicating better adhesion or high irritation (low, medium, high).

Results -

Table 1 shows the results of testing of the compositions based on an exemplary EPDM, Royalene 521. Examples 1 and 2 demonstrate that the EPDM based hydrocolloid compositions provide good saline absorption, good adhesion to skin and better integrity than Example 3, a PIB based composition found in Table 2.

Exemplary embodiments of hydrocolloid formulations may be comprised of up to about 70% EPDM, up to about 95% PIB, up to about 35% tackifier, up to about 35% plasticizers and up to about 40% hydrophilic particles. Other embodiments may not include PIB, as seen in Table 3, composition 16. Additional exemplary hydrocolloid compositions having EPDM may or may not include up to about 30 % SBC, as also shown in Table 3, compositions 17 and 18. Furthermore, exemplary hydrocolloid compositions made according to the teachings of the present invention may also further comprise up to about 5% stabilizers.

**Table 1. EPDM based compositions**

| **Composition** | | **1** | **2** |
|---|---|---|---|
| | | **%ages** | **%ages** |
| EDPM | (Royalene 521) | **21** | **21** |
| PIB | (Parapol 1300) | **17** | **17** |
| | (Eastotac 100L) | **21** | **21** |
| | (Eastoflex E1003) | - | **11** |
| | (Eastoflex E1060) | **11** | - |
| Hydrophilic particles | | **30** | **30** |
| (sodium carboxymethylcellulose) | | | |
| | | | |
| % saline Absorption | | **150** | **221** |
| Observed Integrity | | **8** | **9** |
| Skin Adhesion | | **6** | **7** |

Table 2 shows the results of testing nine examples of hydrocolloid compositions. Example 3: a pure PIB hydrocolloid composition per U.S. Pat. 3,339,546; Example 4-7: based on the practice described in U.S. Pat. 4,551,490, using butyl rubber to enhance the integrity of the PIB hydrocolloid compositions. Although butyl rubber (Butyl 268 and Butyl 269) enhanced the integrity of the compositions, the absorption of the modified systems was not as high as the EPDM systems, and adhesion to skin were also slightly lower than the EPDM formulations. Examples 8-11 are exemplary hydrocolloid compostions according to the teachings of the present invention. EPDM based hydrocolloid compositions maintained high absorption, and enhanced integrity and brought the rating up to 8 - 9 in a scale 10. All of the compositions showed low irritation to skin.

**Table 2. Previous PIB Based Compositions (3-7) and Exemplary EPDM Containing Compositions (8-12)**

| | U.S. Pat. | | U.S. Pat. | | | Preferred Hydrocolloid | | | |
|---|---|---|---|---|---|---|---|---|---|
| | Compositions | | | | | | | | |
| | 3,339,564 ← ← 4,551,490 → → | | | | | | | | |
| **Composition** | **3** | **4** | **5** | **6** | **7** | **8** | **9** | **10** | **11** |
| | % | % | % | % | % | % | % | % | % |
| EPDM (Royalene 521) | - | - | - | - | - | **9** | **8.6** | **8.4** | **15** |
| PIB (PIB 6H) | 68 | 56 | 50 | 56 | 50 | **59** | **58** | **56** | **50** |
| Plasticizer (Parapol 1300) | 2 | 5.6 | 5 | 5.6 | 5 | **2** | **3.4** | **5.6** | **5** |
| Butyl Rubber (Butyl 268) | - | 8.4 | 15 | - | - | **-** | **-** | **-** | **-** |
| (Butyl 269) | - | - | - | 8.4 | 15 | **-** | **-** | **-** | **-** |
| | | | | | | | | | |
| Hydrophilic particles | 30 | 30 | 30 | 30 | 30 | **30** | **30** | **30** | **30** |
| (sodium carboxymethylcellulose) | | | | | | | | | |
| % Saline Absorption | 363 | 245 | 229 | 251 | 209 | **351** | **328** | **318** | **350** |
| Observed Integrity | 1 | 9 | 9 | 9 | **9** | **8** | **9** | **8** | **8** |
| Skin Adhesion | 5 | 7 | 5 | 5 | 5 | **7** | **6** | **7** | **9** |
| Skin Irritation | low | low | low | low | low | **low** | **low** | **Low** | **low** |

Table 3 shows the results of testing an additional seven novel and exemplary hydrocolloidal compositions (Examples 12-18). The EPDM rubber utilized in these tests was Royalene 512, which has a higher molecular weight than the Royalene 521 utilized in the experiments summarized in Table 1 and 2. As mentioned previously, Royalene 512 provides better cohesion than Royalene 521. Composition #16 demonstrates that Royalene 512 (an exemplary EPDM), when compounded with tackification ingredients and without PIB or other rubbers, provides good adhesion to skin and resistance to saline. Composition #15 shows that Royalene 512 mixed with PIB and a tackifier, provides a better balance of adhesion, absorption and integrity.

The results summarized in Table 3 also show that the teachings of the present invention provide hydrocolloidal compositions comprised of EPDM which work well with blends of hydrocolloid materials like Xanthan Gum and sodium carboxymethylcellulose (CMC) (Composition #12 - #18), in addition to the neat sodium CMC, utilized as an exemplary hydrophilic particle component in compositions listed in Table 1 and Table 2.

Furthermore, hydrocolloid compositions comprised of EPDM also work well with other high cohesion rubbers such as styrenic block copolymer rubber (SBC). Composition #17 and #18 are examples utilizing Vector 4113, a tri-block SIS rubber, which achieve a balance of good skin adhesion, saline absorption and integrity. EPDM can be mixed with other polymers and rubbers as a part of a pressure sensitive adhesive phase. That is, in a hydrocolloid formula, the continuous phase of the rubber adhesive, the non-hydrocolloid powder phase, is called adhesive phase, continuous phase, or pressure sensitive adhesive phase since it is a selftack material, as known to those skilled in the art. The other useful rubbers and polymers include, but are not limit to, butyl rubbers, styrene butadiene rubbers (SBR), natural rubber and ethylene vinyl acetate copolymers (EVA), as well as others.

**Table 3. Results Utilizing Exemplary EPDM Containing Compositions**

| **Composition #** | **12** | **13** | **14** | **15** | **16** | **17** | **18** |
|---|---|---|---|---|---|---|---|
| | % | % | % | % | % | % | % |
| EPDM (Royalene 512) | 13 | 36 | 28 | 23 | 23 | 32 | 7 |
| PIB (Vistanex LM-MH) | 29 | 18 | 14 | 23 | | 16 | 14 |
| SBC (Vector 4113) | | | | | | 8 | 28 |
| Plasticizer (Parapol 1300) | 6 | 4 | 14 | | 23 | 3 | 10 |
| Tackifier (Escorez 1310LC) | 21 | 12 | 14 | 23 | 23 | 11 | 10 |
| Stabilizer (Irganox 1010) | 1 | 1 | 1 | 1 | 1 | 1 | 1 |
| | | | | | | | |
| Hydrophilic particles | | | | | | | |
| Xanthan Gum | 24 | 24 | 24 | 24 | 24 | 24 | 24 |
| Sodium CMC | 6 | 6 | 6 | 6 | 6 | 6 | 6 |
| | | | | | | | |
| % Saline Absorption | 251 | 90 | 76 | 224 | 71 | 141 | 170 |
| Observed Integrity (0-10) | 6 | 10 | 10 | 10 | 10 | 10 | 10 |
| Skin Adhesion (0-10) | | 1 | 4 | 8 | 7 | 7 | 6 |
| Skin Irritation | Low | Low | Low | Low | Low | Low | Low |

These examples show that EPDM alone (Composition 16), or in combination with PIB provides enhanced integrity of hydrocolloid compositions, and improves the adhesion of such compositions to skin as well as maintains the high saline absorption feature. Furthermore, compounds formulated according to the teachings of the present invention minimizes irritation to the skin.

Additional exemplary hydrocolloid compositions may also be comprised of EPDM and SBC, as seen in the exemplary formulations that follow. Exemplary EPDM and SBC combinations may be, for example, about 20 parts EPDM and about 80 parts SBC (totaling 100 parts). Additional components may include tackifiers from about 60 to about 200 parts and may also include plasticizer from about 0 to 200 parts. Further components may also include from about 20 parts to 500 parts of hydrophilic particles.

Ultra-high molecular weight PIB may also be utilized to provide exemplary hydrocolloid compositions, according to the teachings of the present invention. For example, the MW of these ultra-high molecular weight PIBs may be on the order of about 100,000 to 1.5 million Dalton.

It is also further contemplated that EPDM may be blended with other components to provide novel and improved hydrocolloid compositions. Examples of these components include nitrile rubber (such as poly(acryl nitrile-co-butadiene, for example), poly-isoprenes, polyurethane, halobutyl rubbers, amorphous poly-co-alpha olefins, EVA (ethylene vinyl acetate) and SBR (styrene butadiene rubbers).

## Claims

1. A hydrocolloid composition comprising an ethylene propylene diene monomer polymer including 25% to 75% ethylene and 75% to 25% propylene, hydrophilic particles and a stabilizer, wherein the stabilizer is butyl rubber.

2. The hydrocolloid composition according to claim 1 further comprising an ethylene propylene diene monomer polymer and a polyisobutylene

3. The hydrocolloid composition according to claim 1 further comprising an ethylene propylene diene monomer polymer, a polyisobutylene and an ethylene propylene elastomer.

4. The hydrocolloid composition according to any one of claims 2 and 3, comprising 5 to 70% ethylene propylene diene monomer, and 95% to 0% polyisobutylene.

5. The hydrocolloid composition according to any one of claims from 2 to 4, where in the polyisobutylene has a molecular weight of 36,000 to 100,000.

6. The hydrocolloid composition according to claim 5 where in the polyisobutylene has a molecular weight of 45000 to 60000.

7. The hydrocolloid composition according to any one of the preceding claims further hydrophilic particles.

8. The hydrocolloid composition according to claim 7, wherein the hydrophilic particles comprise 20% to 40% of the composition.

9. The hydrocolloid composition according to any one of the preceding claims further comprising therapeutic agents.

10. The hydrocolloid composition according to any one of the preceding claims further comprising additives.

11. The hydrocolloid composition according to claim 10 wherein the additives are selected from the group consisting of tackifiers, hydrophilic particles and plasticizers.

12. The hydrocolloid composition according to any one of the preceding claims further comprising a pigment.

13. The hydrocolloid composition according to claim 1 comprising 5% to 40% EDPM, 10% to 80% PIB, up to 35% plasticizer, up to 35% tackifier, and up to 40% hydrophilic particles.

14. The hydrocolloid composition according to claim 1 further comprising a polyisobutylene and/or a styrenic block copolymer.

15. The hydrocolloid composition according to claim 14 wherein said styrenic block copolymer comprises; up to 35% of the hydrocolloid composition.

16. The hydrocolloid composition according to claim 15 further comprising additives.

17. The hydrocolloid composition according to claim 16 wherein the additives are selected from the group consisting of tackifiers, hydrophilic particles and plasticizers.

18. A dressing comprising:
a hydrocolloid composition according to any one of the preceding claims; and
a substrate with the hydrocolloid composition disposed thereon.

19. The dressing of claim 18 further comprising additives.

20. The dressing of claim 19 wherein the additives are selected from the group consisting of tackifiers, stabilizers, hydrophilic particles, therapeutic agents and plasticizers.

21. The dressing according to any one of claims 18 to 20 wherein the dressing is in the form of a wound dressing, ostomy dressing, bandage or blister patch.

## Patentansprüche

1. Hydrokolloidzusammensetzung mit einem Ethylen-Propylen-Dienmonomer-Polymer umfassend 25% bis 75% Ethylen und 75% bis 25% Propylen, hydrophilen Partikeln und einem Stabilisator, **dadurch gekennzeichnet, dass** der Stabilisator Butylkautschuk ist.

2. Hydrokolloidzusammensetzung gemäß Anspruch 1, die zusätzlich ein Ethylen-Propylen-Dienmonomer-Polymer und ein Polyisobutylen umfasst.

3. Hydrokolloidzusammensetzung gemäß Anspruch 1, die zusätzlich ein Ethylen-Propylen-Dienmonomer-Polymer, ein Polyisobutylen und ein Ethylen-Propylen-Elastomer umfasst.

4. Hydrokolloidzusammensetzung gemäß Anspruch 2 oder 3, die 5 bis 70% Ethylen-Propylen-Dienmonomer und 95% bis 0% Polyisobutylen umfasst.

5. Hydrokolloidzusammensetzung gemäß mindestens einem der Ansprüche 2 bis 4, **dadurch gekennzeichnet, dass** das Polyisobutylen ein Molekulargewicht von 36.000 bis 100.000 aufweist.

6. Hydrokolloidzusammensetzung gemäß Anspruch 5, **dadurch gekennzeichnet, dass** das Polyisobutylen ein Molekulargewicht von 45000 bis 60000 aufweist.

7. Hydrokolloidzusammensetzung gemäß mindestens einem der vorhergehenden Ansprüche, die zusätzlich hydrophile Partikel umfasst.

8. Hydrokolloidzusammensetzung gemäß Anspruch 7, **dadurch gekennzeichnet, dass** die hydrophilen Partikel 20% bis 40% der Zusammensetzung bilden.

9. Hydrokolloidzusammensetzung gemäß mindestens einem der vorhergehenden Ansprüche, die zusätzlich Therapeutika umfasst.

10. Hydrokolloidzusammensetzung gemäß mindestens einem der vorhergehenden Ansprüche, die zusätzlich Additive umfasst.

11. Hydrokolloidzusammensetzung gemäß Anspruch 10, **dadurch gekennzeichnet, dass** die Additive ausgewählt sind aus der Gruppe bestehend aus Klebrigmacher, hydrophilen Partikel und Weichmacher.

12. Hydrokolloidzusammensetzung gemäß mindestens einem der vorhergehenden Ansprüche, die zusätzlich ein Pigment umfasst.

13. Hydrokolloidzusammensetzung gemäß Anspruch 1, die 5% bis 40% EDPM, 10% bis 80% PIB, bis zu 35% Weichmacher, bis zu 35% Klebrigmacher und bis zu 40% hydrophile Partikel umfasst.

14. Hydrokolloidzusammensetzung gemäß Anspruch 1, die zusätzlich ein Polyisobutylen- und/oder ein Styrol- Blockcopolymer umfasst.

15. Hydrokolloidzusammensetzung gemäß Anspruch 14, **dadurch gekennzeichnet, dass** das Styrol- Blockcopolymer bis zu 35% der Hydrokolloidzusammensetzung bildet.

16. Hydrokolloidzusammensetzung gemäß Anspruch 15, die zusätzlich Additive umfasst.

17. Hydrokolloidzusammensetzung gemäß Anspruch 17, **dadurch gekennzeichnet, dass** die Additive ausgewählt sind aus der Gruppe bestehend aus Klebrigmacher, hydrophilen Partikel und Weichmacher.

18. Ein Verband umfassend:
eine Hydrokolloidzusammensetzung gemäß mindestens einem der vorhergehenden Ansprüche und
ein Substrat auf dem die Hydrokolloidzusammensetzung verteilt ist.

19. Verband gemäß Anspruch 18, der zusätzlich Additive umfasst.

20. Verband gemäß Anspruch 19, **dadurch gekennzeichnet, dass** die Additive ausgewählt sind aus der Gruppe bestehend aus Klebrigmacher, hydrophilen Partikel und Weichmacher.

21. Verband gemäß mindestens einem der Ansprüche 18 bis 20, **dadurch gekennzeichnet, dass** der Verband ein Wundverband, ein Stomaverband, eine Bandage oder ein Blasenpflaster ist.

## Revendications

1. Composition hydrocolloïde comprenant un polymère formé à partir d'un monomère d'éthylène propylène diénique incluant 25% à 75% d'éthylène et 75% à 25% de propylène, des particules hydrophiles et un stabilisant, dans laquelle le stabilisant est le butylcaoutchouc.

2. Composition hydrocolloïde selon la revendication 1 comprenant de plus un polymère formé à partir d'un monomère d'éthylène propylène diénique et un polyisobutylène.

3. Composition hydrocolloïde selon la revendication 1 comprenant de plus un polymère formé à partir d'un monomère d'éthylène propylène diénique, un polyisobutylène et un élastomère propylène éthylène.

4. Composition hydrocolloïde selon l'une quelconque des revendications 2 et 3, comprenant 5 à 70% du monomère d'éthylène propylène diénique et 95% à 0% de polyisobutylène.

5. Composition hydrocolloïde selon l'une quelconque des revendications 2 à 4, dans laquelle le polyisobutylène a un poids moléculaire de 36000 à 100000.

6. Composition hydrocollo'ide selon la revendication 5, dans laquelle le polyisobutylène a un poids moléculaire de 45000 à 60000.

7. Composition hydrocolloïde selon l'une quelconque des revendications précédentes comprenant de plus des particules hydrophiles.

8. Composition hydrocolloïde selon la revendication 7, dans laquelle les particules hydrophiles constituent 20% à 40% de la composition.

9. Composition hydrocolloïde selon l'une quelconque des revendications précédentes comprenant de plus des agents thérapeutiques.

10. Composition hydrocolloïde selon l'une quelconque des revendications précédentes comprenant de plus des additifs.

11. Composition hydrocolloïde selon la revendication 10, dans laquelle les additifs sont sélectionnés à partir du groupe constitué d'agents poisseux, des particules hydrophiles et de plastifiants.

12. Composition hydrocolloïde selon l'une quelconque des revendications précédentes comprenant de plus un pigment.

13. Composition hydrocolloïde selon la revendication 1 comprenant 5% à 40% d'EDPM, 10% à 80% de PIB, jusqu'à 35% de plastifiant, jusqu'à 35% d'agent poisseux, et jusqu'à 40% de particules hydrophiles.

14. Composition hydrocolloïde selon la revendication 1 comprenant de plus un copolymère bloc styrénique et/ou un polyisobutylène.

15. Composition hydrocolloïde selon la revendication 14 dans laquelle le copolymère bloc styrénique constitue jusqu'à 35% de la composition hydrocolloïde.

16. Composition hydrocolloïde selon la revendication 15 comprenant de plus des additifs.

17. Composition hydrocolloïde selon la revendication 16 dans laquelle les additifs sont sélectionnés à partir du groupe constitué d'agents poisseux, de particules hydrophiles et de plastifiants.

18. Pansement comprenant :
une composition hydrocolloïde selon l'une quelconque des revendications précédentes ; et
un substrat sur lequel est disposée la composition hydrocolloïde.

19. Pansement selon la revendication 18 comprenant de plus des additifs.

20. Pansement selon la revendication 19, dans lequel les additifs sont sélectionnés à partir du groupe constitué d'agents poisseux, de stabilisants, de particules hydrophiles, d'agents thérapeutiques et de plastifiants.

21. Pansement selon l'une quelconque des revendications 18 à 20, dans lequel le pansement est sous la forme d'un pansement pour plaie, d'un pansement pour stomie, d'un bandage ou d'un timbre cutané alvéolé.
